(19) Europäisches Patentamt
European Patent Office
Office européen des brevets

(11) EP 1 452 523 A2

(12) **EUROPEAN PATENT APPLICATION**

(43) Date of publication:
**01.09.2004 Bulletin 2004/36**

(51) Int Cl.⁷: **C07C 381/10**

(21) Application number: **04250921.6**

(22) Date of filing: **20.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL HR LT LV MK**

(30) Priority: **25.02.2003 JP 2003047744**
**02.09.2003 JP 2003310132**

(71) Applicant: **KYUSHU UNIVERSITY**
**Fukuoka City Fukuoka Pref. (JP)**

(72) Inventors:
- **Katsuki, Tsutomu**
  **Fukuoka City Fukuoka Pref (JP)**
- **Tamura, Yusuke**
  **Dazaifu City Fukuoka Pref. (JP)**
- **Uchida, Tatsuya**
  **Fukuoka City Fukuoka Pref. (JP)**

(74) Representative: **Whalley, Kevin**
**MARKS & CLERK,**
**57-60 Lincoln's Inn Fields**
**London WC2A 3LS (GB)**

(54) **Method of producing optically active sulfimide compounds**

(57) An optically active sulfimide compound is produced by using a specified Ru(salen)(CO) complex as a catalyst and subjecting a specified alkyl aryl sulfide compound to an asymmetric sulfimidation with a specified azide compound having an easily eliminating group.

EP 1 452 523 A2

## Description

### BACKGROUND OF THE INVENTION

### 1. Field of the Invention

This invention relates to a method of producing an optically active sulfimide compound. Such an optically active sulfimide compound is usable for the synthesis of medicines and agricultural chemicals, and is also useful as a chiral auxiliary in organic synthesis likewise an optically active sulfoxide compound.

### 2. Description of the Related Art

Nitrene transfer reaction is the most important method for the synthesis of optically active compounds including a nitrogen functional group. Therefore, many catalysts have been developed for this reaction and high enantioselectivity has been achieved in many reactions.

However, there is still a big room for improvement in the atom efficiency of the nitrene precursor because N-arylsulfonyliminophenyliodinanes ($ArSO_2N$=IPh) , which are low atom efficient reagents, have been used as nitrene precursors in those reactions. Recently, the use of azide compounds as the nitrene precursors, which produce the corresponding nitrene and the by-product nitrogen, has been examined.

For example, Jacobsen et al. reported that N-arylsulfonyl azide serves as a nitrene precursor for an asymmetric aziridination in the presence of a copper ion under photo-irradiation.

Moreover, the inventors reported that a Ru(salen)(CO) complex catalyzes a sulfimidation of alkyl aryl sulfides using an arylsulfonyl azide as a nitrene precursor without photo-irradiation in a highly enantioselective manner (Murakami M., Uchida T. and Katsuki T., Tetrahedron Letters, 2001, 42, 7071-7074). However, the removal of an arylsulfonyl group from the resulting nitrogen-containing compound usually needs harsh reaction conditions.

### SUMMARY OF THE INVENTION

It is, therefore, an object of the invention to provide a method capable of producing an optically active sulfimide compound with high enantioselectivity by using a compound having an easily eliminating group as a nitrene precursor.

The inventors have made various studies in order to achieve the above object and found that a sulfimide compound having a high optical purity can be produced by using a specified Ru(salen)(CO) complex as a catalyst and subjecting an alkyl aryl sulfide compound to an asymmetric sulfimidation with a specified azide compound having a carbamoyl group, and as a result the invention has been accomplished.

According to the invention, there is the provision of a method of producing an optically active sulfimide compound, which comprises using as a catalyst an optical active Ru(salen)(CO) complex represented by the following formula (I) or (II) and subjecting an alkyl aryl sulfide compound represented by the following formula (III) to an asymmetric sulfimidation with an azide compound represented by the following formula (IV):

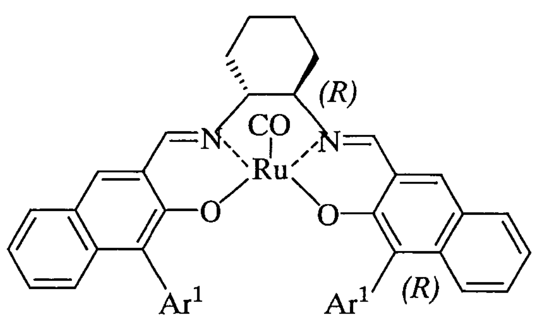

· · · · · (I)

wherein $Ar^1$ is independently an aryl group having a carbon number of 10 to 16;

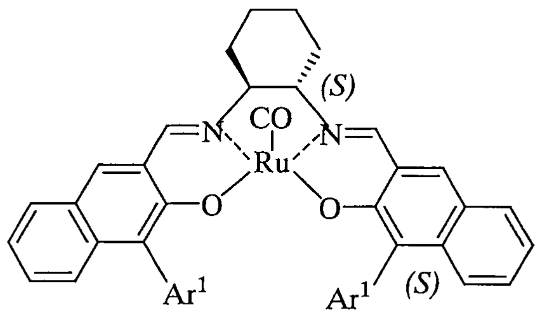

····· (II)

wherein $Ar^1$ is independently an aryl group having a carbon number of 10 to 16;

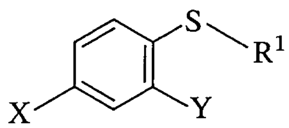

····· (III)

wherein $R^1$ is a straight or branched alkyl group having a carbon number of 1 to 10 and X and Y are independently a hydrogen atom, a halogen atom, a nitro group or an alkoxy group having a carbon number of 1 to 4;

$$R^2\text{-}OCON_3 \qquad (IV)$$

wherein $R^2$ is a straight or branched alkyl group having a carbon number of 1 to 15, an aralkyl group having a carbon number of 7 to 13 or an aryl group having a carbon number of 6 to 10, provided that a hydrogen atom in the alkyl group, the aralkyl group and the aryl group may be substituted with a halogen atom.

In a preferable embodiment of the invention, the Ru(salen)(CO) complex is represented by the following formula (V) or (VI).

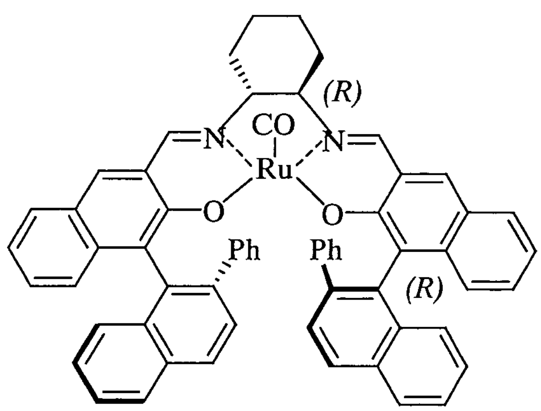

····· (V)

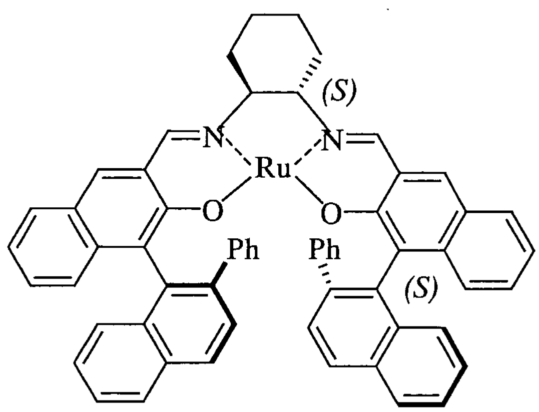

· · · · · (VI)

In another preferable embodiment of the invention, the alkyl aryl sulfide compound of the formula (III) is methyl phenyl sulfide, methyl p-methoxyphenyl sulfide, methyl p-chlorophenyl sulfide, ethyl phenyl sulfide, methyl o-bromophenyl sulfide or methyl o-nitrophenyl sulfide.

In the other preferable embodiment of the invention, $R^2$ in the azide compound of the formula (IV) is methyl group, n-butyl group, benzyl group, t-butyl group, phenyl group, 2,2,2-trichloroethyl group or 2,2,2-trichloro-1,1-dimethylethyl group. As $R^2$ in the formula (IV), 2,2,2-trichloro-1,1-dimethylethyl group is particularly preferable.

In a further preferable embodiment of the invention, the sulfimide compound is represented by the following formula (VII):

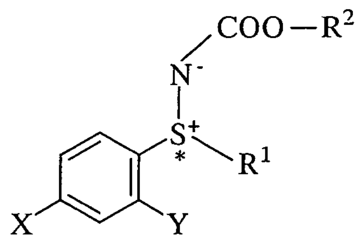

· · · · · (VII)

wherein each of $R^1$, $R^2$, X and Y is the same meaning as mentioned above.

As the sulfimide compound of the formula (VII) may be mentioned a compound wherein $R^1$ is methyl group or ethyl group, X is a hydrogen atom, a chlorine atom or methoxy group, Y is a hydrogen atom, a bromine atom or a nitro group and $R^2$ is methyl group, n-butyl group, benzyl group, t-butyl group, phenyl group, 2,2,2-trichloroethyl group or 2,2,2-trichloro-1,1-dimethylethyl group. Among the sulfimide compounds of the formula (VII), a compound in which $R^2$ is 2,2,2-trichloro-1,1-dimethylethyl group is particularly preferable.

As mentioned above, according to the production method of the invention, the azide compound of the formula (IV) having an easily eliminating $R^2$-OCO group can be used as a nitrene precursor to reduce the cost for removing a protection group from the product. Also, both of optically active sulfimide enantiomers can be produced in a highly enantioselective manner. Thus, the optically active sulfimide compound capable of using as a chiral auxiliary for the synthesis of medicines and agricultural chemicals or the organic synthesis can be provided at a high optical purity.

DETAILED DESCRIPTION OF THE INVENTION

The invention will be explained in detail below. The optically active Ru(salen)(CO) complex used as a catalyst in the invention is represented by the formula (I) or (II). The complex of the formula (II) is an enantiomer of the complex of the formula (I). In the formulae (I) and (II), $Ar^1$ is independently an aryl group having a carbon number of 10 to 16. As the aryl group having a carbon number of 10 to 16, mention may be made of 1-naphthyl group, 2-biphenyl group, 2-phenyl-1-naphthyl group, 2-methyl-1-naphthyl group, 2-(3,5-dimethylphenyl)-1-naphthyl group, 2-(4-methylphenyl)-1-naphthyl group, 2-[4-(t-butyldiphenylsilyl)-phenyl]-1-naphthyl group, 2-methoxy -1-naphthyl group and so on. Among them, 2-phenyl-1-naphthyl group is particularly preferable as $Ar^1$ in view of catalytic activity and enantioselectivity. In the latter case, the Ru(salen)(CO) complex is represented by the formula (V) or (VI). The Ru(salen)(CO) complex has a CO ligand at its apical position. An amount of the Ru(salen)(CO) complex used as a catalyst is a range of 0.1 to 100

mol%, preferably 1 to 4 mol% per the molar amount of the alkyl aryl sulfide as a substrate.

The alkyl aryl sulfide used in the invention is represented by the formula (III). In the formula (III), $R^1$ is a straight or branched alkyl group having a carbon number of 1 to 10. As the alkyl group having a carbon number of 1 to 10, mention may be made of methyl group, ethyl group, propyl group, isopropyl group, butyl group, isobutyl group, t-butyl group, pentyl group, isopentyl group, neopentyl group, hexyl group, heptyl group, octyl group, 2-ethylhexyl group, nonyl group, decyl group and so on. The alkyl group may be substituted with a halogen or the like. Further, X and Y are independently a hydrogen atom, a halogen atom, a nitro group or an alkoxy group having a carbon number of 1 to 4. As the alkoxy group having a carbon number of 1 to 4, mention may be made of methoxy group, ethoxy group, propoxy group, butoxy group and so on. The alkoxy group may be substituted with a halogen and so on.

Concretely, the alkyl aryl sulfide of the formula (III) includes methyl phenyl sulfide, methyl p-methoxyphenyl sulfide, methyl p-chlorophenyl sulfide, ethyl phenyl sulfide, methyl o-bromophenyl sulfide, methyl o-nitrophenyl sulfide, methyl p-nitrophenyl sulfide, methyl p-bromophenyl sulfide and so on. However, when a dialkyl sulfide is used as the sulfide compound, enantioselectivity is undesirably low.

The azide compound used as a nitrene precursor in the invention is a N-carbamoyl azide represented by the formula (IV), which has an easily eliminating $R^2$-OCO group. In the formula (IV), $R^2$ is a straight or branched alkyl group having a carbon number of 1 to 15, an aralkyl group having a carbon number of 7 to 13 or an aryl group having a carbon number of 6 to 10, provided that a hydrogen atom in the alkyl group, the aralkyl group and the aryl group may be substituted with a halogen atom. As the straight or branched alkyl group having a carbon number of 1 to 15, mention may be made of methyl group, ethyl group, n-butyl group, t-butyl group, isopropyl group, bis(cyclohexyl)methyl group and so on. As the alkyl group substituted with the halogen atom, mention may be made of 2,2,2-trichloroethyl group, 2,2,2-trichloro-1,1-dimethylethyl group and so on. As the aralkyl group having a carbon number of 7 to 13, mention may be made of benzyl group, 2-phenylethyl group, 3-phenylpropyl group, diphenylmethyl group and so on. As the aryl group having a carbon number of 6 to 10, mention may be made of phenyl group, tolyl group, 2,4,6-trimethylphenyl group and so on. Among them, as $R^2$ in the formula (IV), the alkyl group and aralkyl group are preferable, and the alkyl group substituted with the halogen is particularly preferable. These N-carbamoyl azides can be easily synthesized, for example, by treating commercially available 2,2,2-trichloroethyl chloroformate or 2,2,2-trichloro-1,1-dimethylethyl chloroformate with sodium azide in acetone and are easily available.

An amount of the azide compound of the formula (IV) used is a range of 1 to 3 equivalent, preferably 1 to 1.5 equivalent to the above-mentioned alkyl aryl sulfide compound. From a viewpoint of enhancing the yield, it is preferable that the molar amount of the azide compound is made somewhat excess as compared with that of the alkyl aryl sulfide compound.

The final product according to the invention, i.e. optically active sulfimide compound is represented by the formula (VII). Moreover, both of optically active sulfimide enantiomers are obtained by properly using the Ru(salen)(CO) complex of the formula (I) and the Ru(salen)(CO) complex of the formula (II). In the formula (VII), each of $R^1$, $R^2$, X and Y is the same meaning as mentioned above. Such an optically active sulfimide compound is usable for the synthesis of medicines and agricultural chemicals, and is also useful as a chiral auxiliary in the organic synthesis likewise an optically active sulfoxide compound.

The asymmetric sulfimidation conducted in the invention is an enantioselective reaction between a sulfide and a nitrene precursor, which is an asymmetric imidation of the sulfide. In the conventional sulfimidation, an arylsulfonyl azide is used as a nitrene precursor and hence it is difficult to remove an arylsulfonyl group from the resulting product. On the contrary, according to the invention, the azide compound of the formula (IV) having an easily eliminating $R^2$-OCO group is used as a nitrene precursor, so that it is easy to remove the $R^2$-OCO group from the resulting product.

The optical purity, i.e. enantiomeric excess of the sulfimide compound obtained according to the production method of the invention is represented by the following equation:

$$\text{Enantiomeric excess (\%ee)} = \frac{[\alpha]_D \times 100}{[\alpha]_{Dmax}} = \frac{(R - S) \times 100}{R + S} \text{ or } \frac{(S - R) \times 100}{R + S}$$

wherein $[\alpha]_D$ is a specific rotation of a sample, $[\alpha]_{Dmax}$ is a specific rotation of an optically pure substance, R is a ratio of R-isomer occupied in the sample, and S is a ratio of S-isomer occupied in the sample. When the ratios of R-isomer and S-isomer are the same, that is, when the sample is a racemic modification, the enantiomeric excess is 0 %ee. The enantiomeric excess of the product can be measured by means of a high performance liquid chromatography (HPLC) using an optically active column.

In the invention, it is preferable to conduct the sulfimidation in the presence of a zeolite. Although the sulfimidation proceeds in a good enatioselective manner without the zeolite, the yield can be largely improved by conducting the reaction in the presence of the zeolite. As the zeolite used in the invention, mention may be made of MS-3A, MS-4A, MS-5A and so on. Among them, MS-4A is preferable. An amount of the zeolite used is a range of 50 to 500 mg,

preferably 100 to 300 mg per 1 mmol of the alkyl aryl sulfide compound as a substrate.

The asymmetric sulfimidation is carried out in a solvent. As the solvent, mention may be made of chlorobenzene, dichloroethane, toluene, dichloromethane and so on. Among them, dichloromethane is preferable. An amount of the solvent used is a range of 2 to 50 mL, preferably 4 to 10 mL per 1 mmol of the alkyl aryl sulfide as a substrate.

The production method of the optically active sulfimide compound according to the invention may be carried out at 10 to 40°C, preferably at room temperature. Since such a method can be carried out at room temperature under moderate temperature conditions, an energy cost for the temperature adjustment can be suppressed. In the invention, the optically active sulfimide compound can be produced by agitating a mixed solution of the alkyl aryl sulfide compound, the azide compound, the solvent and the catalyst. The reaction time is not particularly limited and is properly selected in accordance with the reaction temperature. It is preferable that when the reaction temperature is high, the reaction time is short, while when the reaction temperature is low, the reaction time is long. However, the reaction proceeds very slowly as the reaction temperature is lowered to 0°C.

The following examples are given in illustration of the invention and are not intended as limitations thereof.

(Synthesis Example 1 of Complex)

(1R, 2R)-1,2-diaminocyclohexane [made by Aldrich Chem. Co.] (27.5 mg, 0.24 mmol) is dissolved into ethanol (5 mL). To the resulting solution is added (aR)-3-formyl-2-hydroxy-2'-phenyl-1,1'-binaphthyl [synthesized according to the method described in H. Sasaki, R. Irie, T. Hamada, K. Suzuki and T. Katsuki, Tetrahedron, 50(41), 11827-11838 (1994) or the like] (179.7 mg, 0.48 mmol), and stirred at room temperature for 24 hours. After the completion of the reaction, the resulting precipitates are filtered and dried at 50°C under a reduced pressure for 1 hour. To the dried precipitates are added triruthenium dodecacarbonyl [made by Aldrich Chem. Co.] (152.1 mg, 0.24 mmol) and a dehydrated ethanol (10 mL) under a nitrogen atmosphere, and the resulting suspension is refluxed under heating for 5 days. After the temperature is turned to room temperature, the resulting reaction mixture is concentrated on a rotary evaporator to remove the solvent. The resulting residue is purified with a silica gel column using dichloromethane/ethanol (=20/1) as a developing solvent to obtain a Ru(salen)(CO) complex (80.5 mg, yield: 36%) represented by the formula (V).

The elementary analysis of the thus obtained complex shows H: 4.32%, C: 65.12% and N: 2.35%, which are well coincident with theoretical values of $C_{61}H_{44}N_2O_3Ru \cdot 2H_2O \cdot 2CH_2Cl_2$ (H: 4.52%, C: 65.23%, N: 2.42%). As a result of IR (KBr) measurement of the thus obtained complex, signals inherent to the complex are observed at 1323.1, 1423.4, 1490.9, 1541.0, 1577.7, 1612.4, 1934.5 and 2019.3 $cm^{-1}$.

(Example 1)

The complex of the formula (V) (1.9 mg, 2.0 μmol) is dissolved in dry toluene (1 mL), concentrated azeotropically under vacuum, and re-dissolved in dichloromethane (0.5 mL). To this solution are added methyl phenyl sulfide (11.7 μL, 0.1 mmol) and MS-4A (20 mg), and the resulting suspension is stirred at room temperature for 0.5 hour. To this suspension is then added N-methoxycarbonyl azide (10.9 μL, 0.13 mmol), and the resulting mixture is further stirred at room temperature for 24 hours. After the completion of the reaction, the reaction mixture is chromatographed on silica gel with ethyl acetate to obtain the corresponding sulfimide compound (4.1 mg, yield: 21 %). The enantiomeric excess of the sulfimide compound is 13 %ee as measured by a high performance liquid chromatography (HPLC) using a DAICEL CHIRALPAK AD-H column and a mixture of hexane/isopropanol (=9/1). The results are shown in Table 1.

(Examples 2 - 7)

The same procedure as in Example 1 is repeated except that N-n-butoxycarbonyl azide (0.13 mmol) is used in Example 2, N-benzyloxycarbonyl azide (0.13 mmol) is used in Example 3, N-t-butoxycarbonyl azide (0.13 mmol) is used in Example 4, N-phenyloxycarbonyl azide (0.13 mmol) is used in Example 5, N-2,2,2-trichloroethoxycarbonyl azide (0.13 mmol) is used in Example 6, N-2,2,2-trichloro-1,1-dimethylethoxycarbonyl azide (0.13 mmol) is used in Example 7 instead of N-methoxycarbonyl azide (0.13 mmol). The results are shown in Table 1.

Table 1

| | $R^2$ in azide of formula (IV) | Enantiomeric excess (%ee) | Yield (%) |
|---|---|---|---|
| Example 1 | $CH_3$ | 13 | 21 |
| Example 2 | n-$C_4H_9$ | 18 | 18 |
| Example 3 | $C_6H_5CH_2$ | 36 | 28 |

Table 1 (continued)

| | $R^2$ in azide of formula (IV) | Enantiomeric excess (%ee) | Yield (%) |
|---|---|---|---|
| Example 4 | $t\text{-}C_4H_9$ | 71 | 22 |
| Example 5 | $C_6H_5$ | 8 | 37 |
| Example 6 | $Cl_3CCH_2$ | 77 | 62 |
| Example 7 | $Cl_3CC(CH_3)_2$ | 95 | 93 |

The reaction scheme corresponding to Examples 1 - 7 in Table 1 is shown as follows.

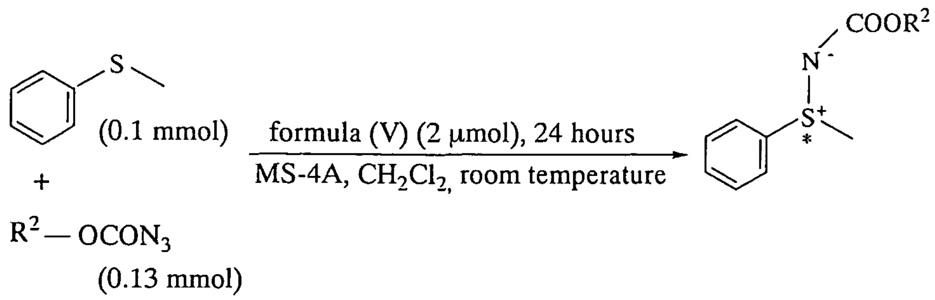


As seen from the results of Table 1, when $R^2$ in the azide compound of the formula (IV) is an alkyl group, the enantioselectivity increases as the bulkiness of the alkyl group becomes larger. From this fact, it is clear that $R^2$ in the azide compound of the formula (IV) affects the enantioselectivity through a steric factor. Also, it is understood that the yield and the enantiomeric excess are further enhanced by substituting a hydrogen of the alkyl group with a chlorine. This is considered due to the fact that $R^2$ in the azide compound of the formula (IV) affects the reaction rate through an electronic factor. Particularly, 2,2,2-trichloro-1,1-dimethylethoxycarbonyl azide having a bulky and electron-withdrawing alkyl group is particularly preferable as the azide compound in the method of the invention.

(Reference Examples 1 - 4)

The same procedure as in Example 1 is repeated except that azide compounds shown in Table 2 (0.13 mmol) are used instead of N-methoxycarbonyl azide (0.13 mmol). The results are shown in Table 2.

Table 2

| | Azide compound | Yield (%) | Enantiomeric excess (%ee) |
|---|---|---|---|
| Reference Example 1 | benzoyl azide | No reaction | |
| Reference Example 2 | P-nitrobenzoyl azide | No reaction | - |
| Reference Example 3 | benzyl azide | No reaction | - |
| Reference Example 4 | p-nitrobenzyl azide | No reaction | - |

As seen from Table 2, no asymmetric sulfimidation occurs in the azide compounds other than the azide compound of the formula (IV).

(Examples 8 -12)

The same procedure as in Example 7 is repeated except that sulfide compounds having structures shown in Table 3 (0.1 mmol) are used instead of methyl phenyl sulfide (0.1 mmol). The results are shown in Table 3.

Table 3

| | Sulfide of formula (III) | | | Enantiomeric excess (%ee) | Yield (%) |
|---|---|---|---|---|---|
| | X | Y | $R^1$ | | |
| Example 7 | H | H | $CH_3$ | 95 | 93 |
| Example 8 | $CH_3O$ | H | $CH_3$ | 96 | 91 |
| Example 9 | Cl | H | $CH_3$ | 95 | 88 |
| Example 10 | H | H | $CH_3CH_2$ | 92 | 87 |
| Example 11 | H | Br | $CH_3$ | 98 | 74 |
| Example 12 | H | $NO_2$ | $CH_3$ | 99 | 99 |

The reaction scheme corresponding to Examples 7 - 12 in Table 3 is shown as follows.

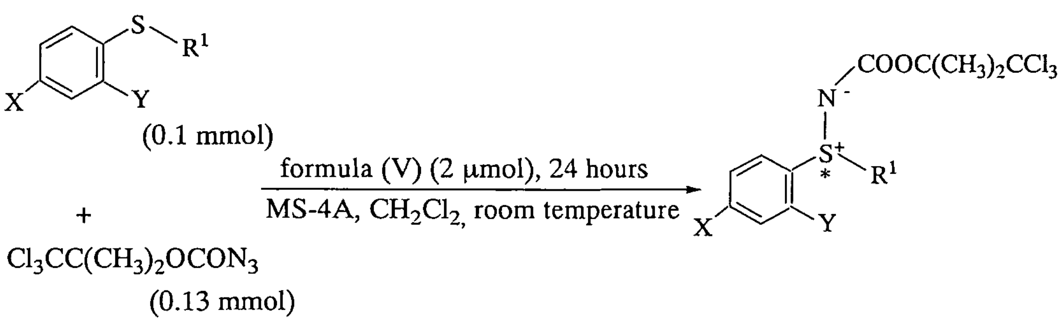


As seen form the results of Table 3, according to the method of the invention, the sulfimidations of various alkyl aryl sulfides can proceed in a highly enantioselective manner.

The optically active sulfimide compounds obtained according to the method of the invention are usable for the synthesis of medicines and agricultural chemicals, and are also useful as a chiral auxiliary in the organic synthesis likewise an optically active sulfoxide compound.

## Claims

1. A method of producing an optically active sulfimide compound, which comprises using as a catalyst an optically active Ru(salen)(CO) complex represented by the following formula (I) or (II) and subjecting an alkyl aryl sulfide compound represented by the following formula (III) to an asymmetric sulfimidation with an azide compound represented by the following formula (IV):

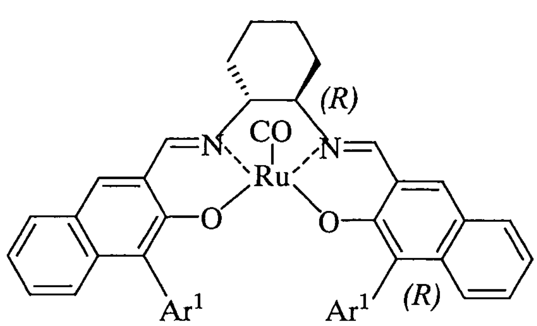

· · · · · (I)

wherein $Ar^1$ is independently an aryl group having a carbon number of 10 to 16;

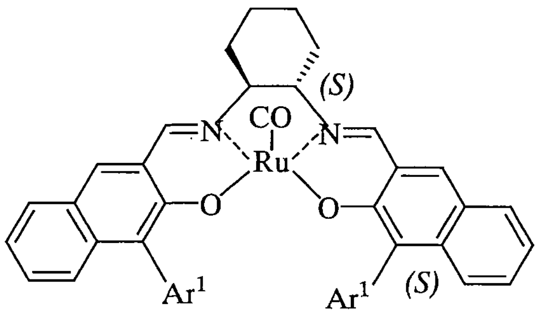

· · · · · (II)

wherein $Ar^1$ is independently an aryl group having a carbon number of 10 to 16;

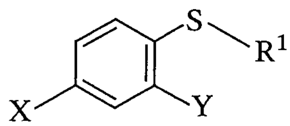

· · · · · (III)

wherein $R^1$ is a straight or branched alkyl group having a carbon number of 1 to 10 and X and Y are independently a hydrogen atom, a halogen atom, a nitro group or an alkoxy group having a carbon number of 1 to 4;

$$R^2\text{-OCON}_3 \qquad \text{(IV)}$$

wherein $R^2$ is a straight or branched alkyl group having a carbon number of 1 to 15, an aralkyl group having a carbon number of 7 to 13 or an aryl group having a carbon number of 6 to 10, provided that a hydrogen atom in the alkyl group, the aralkyl group and the aryl group may be substituted with a halogen atom.

**2.** A method according to claim 1, wherein the Ru(salen)(CO) complex is represented by the following formula (V) or (VI).

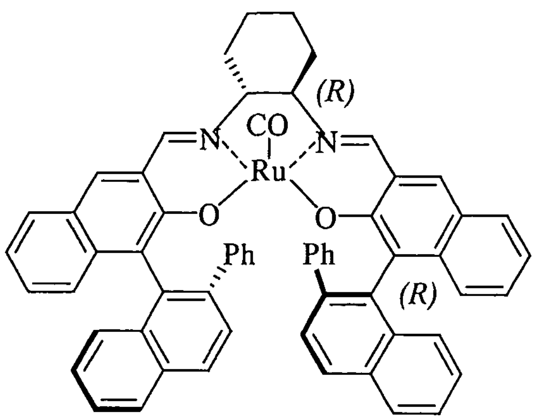

· · · · · (V)

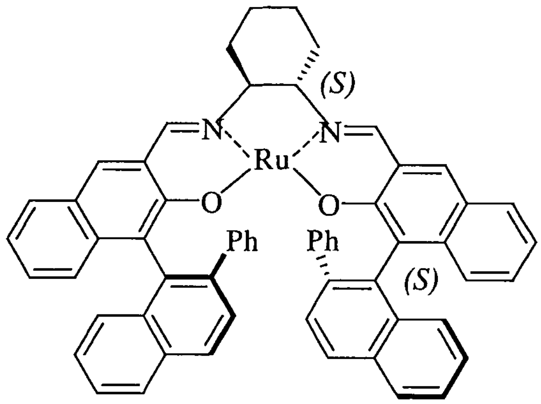

· · · · · (VI)

3. A method according to claim 1, wherein the alkyl aryl sulfide compound of the formula (III) is methyl phenyl sulfide, methyl p-methoxyphenyl sulfide, methyl p-chlorophenyl sulfide, ethyl phenyl sulfide, methyl o-bromophenyl sulfide or methyl o-nitrophenyl sulfide.

4. A method according to claim 1, wherein $R^2$ in the azide compound of the formula (IV) is methyl group, n-butyl group, benzyl group, t-butyl group, phenyl group, 2,2,2-trichloroethyl group or 2,2,2-trichloro-1,1-dimethylethyl group.

5. A method according to claim 4, wherein $R^2$ in the azide compound of the formula (IV) is 2,2,2-trichloro-1,1-dimethylethyl group.

6. A method according to claim 1, wherein the sulfimide compound is represented by the following formula (VII).

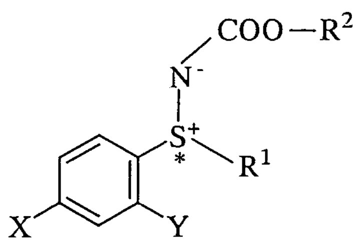

· · · · · (VII)

wherein each of $R^1$, $R^2$, X and Y is the same meaning as mentioned above.

7. A method according to claim 6, wherein $R^1$ is methyl group or ethyl group, X is a hydrogen atom, a chlorine atom or a methoxy group, Y is a hydrogen atom, a bromine atom or a nitro group, and $R^2$ is methyl group, n-butyl group, benzyl group, t-butyl group, phenyl group, 2,2,2-trichloroethyl group or 2,2,2-trichloro-1,1-dimethylethyl group in the sulfimide compound of the formula (VII).

8. A method according to claim 6, wherein $R^2$ in the sulfimide compound of the formula (VII) is 2,2,2-trichloro-1,1-dimethylethyl group.